# EUROPEAN PATENT SPECIFICATION

(11) **EP 1 824 382 B1**
(45) Date of publication and mention of the grant of the patent: **29.08.2012**
(21) Application number: 05801375.6
(22) Date of filing: 19.10.2005
(51) Int. Cl.: A61B 5/026, A61B 5/00

(54) **DVT DETECTION**
DVT-NACHWEIS
DETECTION DVT

(30) Priority: 20.10.2004 GB 0423289
(43) Date of publication of application: 29.08.2007
(73) Proprietor: Huntleigh Technology Limited, Bedfordshire LU1 1TD (GB)
(72) Inventor: GOUGH, Nigel, South Glamorgan CF72 8JT (GB)
(74) Representative: Thaker, Shalini
(86) International application number: PCT/GB2005/004022
(87) International publication number: WO 2006/043052

(56) References cited:
- WO-A-2004/073514
- US-A- 5 090 417
- US-A- 5 282 467
- US-A- 5 991 654

## Description

The present invention relates to the detection of a range of clinical conditions including Deep Vein Thrombosis (DVT) and diabetic peripheral neuropathy, critical limb ischaemia, autonomic neural function and arterial and venous disease by the assessment of the vasomotor activity in the micro-circulation at individual sites on a body, and in particular, the detection of Deep Vein Thrombosis (DVT) and diabetic peripheral neuropathy.

Deep vein thrombosis (DVT) in the legs is a condition whereby a blood clot, develops in a vein causing partial or complete blockage of the vessel. The cause of the clot can be due to vessel damage, either from surgical procedures or trauma, or from a period of haemostasis due to prolonged periods of inactivity (e.g. long haul flight, disability). The perceivable consequences of a DVT can range from mild pain and swelling to a fatal pulmonary embolism.

Known tests used in clinical practices for the detection of DVT include imaging tests such as venography and duplex ultrasonography. Venography requires the injection of a radio opaque imaging medium and X-ray imaging requiring expert interpretation and is hazardous and uncomfortable to the patient, time consuming, expensive and not suitable for primary care or a General Practitioner (GP). Similarly, Duplex ultrasonography is a time consuming and expensive process not suitable for primary care or for GPs requiring highly skilled practitioners.

Plethysmography is a known test which is low cost, relatively quick, and is used in trained primary care or by a trained GP, as shown in WO2004/073514, US5991654 and US5282467. However such plethysmography tests require the patient to exercise during the test which is not suitable for all patients and the test requires an expert operator and is not always reliable. There is also D-dimer assay test that measures the clotting agents in blood and is recommended to be used in conjunction with other tests. The plethysmography and D-dimer tests are used as a front line screening means to remove as many patients as possible without a DVT from progressing to the more onerous imaging tests of duplex ultrasonography or venography.

WO 2004/073514 and US 5,991,654 disclose devices for detecting DVT including a controller, a compression bladder and a light emitter and light receptor placed on a single site of a patient.

The invention seeks to make improvements.

Accordingly, the present invention provides a device as claimed in claim 1.

Vasomotor activity in the micro-circulation is the continuous process of contraction and dilatation of the micro-vessels and serves several important functions including blood pressure regulation, temperature regulation, tissue oxygenation and nutrition. The control of this process is both local and systemic. Local control is activated by chemical signalling from the adjacent tissues while the systemic control originates from the autonomic sympathetic nervous system, principally for the regulation of core temperature and systemic blood pressure. The resulting local blood volume variation provides information on many of the biological processes both locally and systemically.

In a preferred embodiment, the invention comprises a light transmission and detection system including wave transducers, the wave transducers placed at one or more sites on a body, control means to measure the light absorbed and/or reflected at the or more sites and provide signals relating to the absolute value at the or more sites and/or the differential value between the sites. Preferably, the transducers are infra red wave transducers.

The present invention uses the transducers to monitor the micro-circulation blood volume variation beneath the transducer continuously. The light absorption is proportional to the volume of blood or, conversely, light reflection is inversely proportional to blood volume. For a resting patient in a stable environment, either seated or supine, the major changes of blood volume are manifestations of systemic control. Further, in the limbs, the systemic vasomotor control is symmetrical. Therefore, by placing a transducer on the sole of each foot of a healthy subject, the signal from each transducer will be similar if not identical. The presence of a unilateral DVT can be detected by measuring the dissimilarity between the two transducer signals as the distal volume of the affected leg is increased due to increased outflow resistance. This imposes altered frequency and phase characteristics in the vasomotor variation of the affected leg and therefore affects the bilateral symmetry.

In another aspect of the invention the signals received from the transducers are used in the assessment of autonomic systemic and peripheral neuropathy. Conventional systemic, autonomic function testing, analyses heart rate variability, usually derived from the ECG waveform. However, cardiac pulsation can be seen in the signal collected at most points on the skin around the body using the transducer. Therefore, heart rate variability can be derived from this signal. Analysis of the variation in the heart rate component can then be compared to the low frequency variation of the signal from the transducer, allowing a direct comparison of peripheral and systemic autonomic function. In the healthy subject both sources of variation should be similar, whereas in the patient suffering with peripheral neuropathy alone there will be a dissimilarity.

The advantages of using vasomotor activity in the feet to assess DVT, vascular disease and neurological function include the ability to use a passive test requiring no movement on the part of the patient. Preferably, the neurological function test is augmented by stress testing such as valsalva manoeuvre or mild graduation of exhalation impedance. The sites to be used on the patient's body are easily accessible, requiring low cost instruments, lower level of skill than existing tests and providing reliable results.

To date, there is little work published on the use of vasomotor activity for the assessment of clinical conditions such as those of the present invention due to the poor understanding of vasomotor activity and related biological processes. We have found that the vasomotor signal provides valuable information concerning the many biological processes occurring simultaneously within healthy and unhealthy bodies.

The invention will now be described by way of example only, with reference to the following drawings, of which:
Figure 1 shows the light transmission and detection system according to the invention;
Figure 2 shows a block diagram of the transducers in Figure 1;
Figures 3a, b, c are schematic views of a preferred embodiment of the invention in Figure 1 applied to different sites on a patient;
Figure 4 is a signal output from the embodiment as applied in Figure 3a;
Figure 5 shows another preferred embodiment of the invention;
Figure 6 shows the output from the embodiment as shown in Figure 5 from the various sites of the legs of a patient; and
Figure 7 shows the signal response to increased breathing impedance and hand grip.
Figure 8 shows the vasomotor signal and extraction of the heart rate variation.

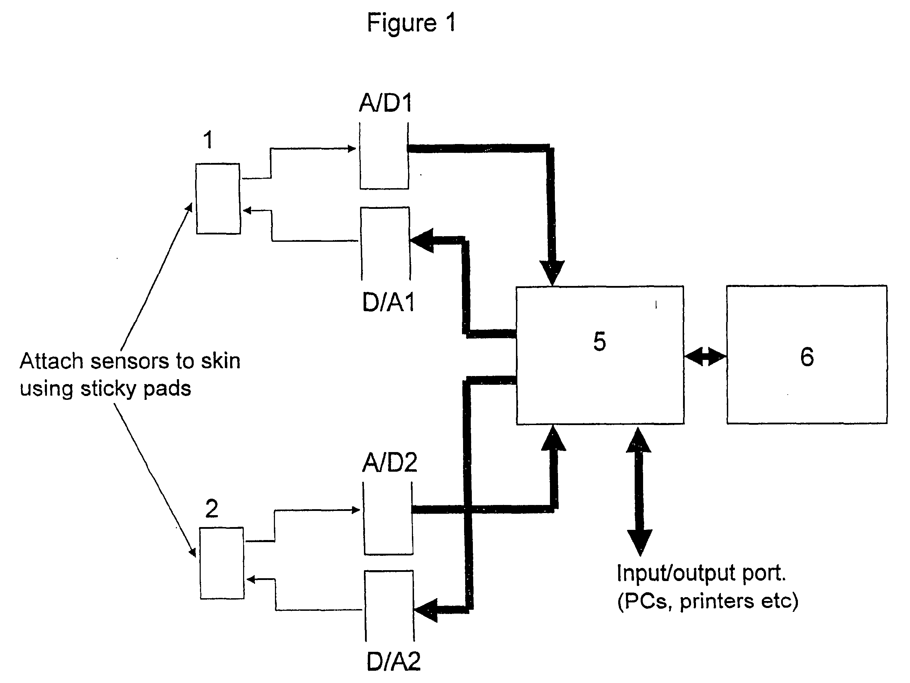

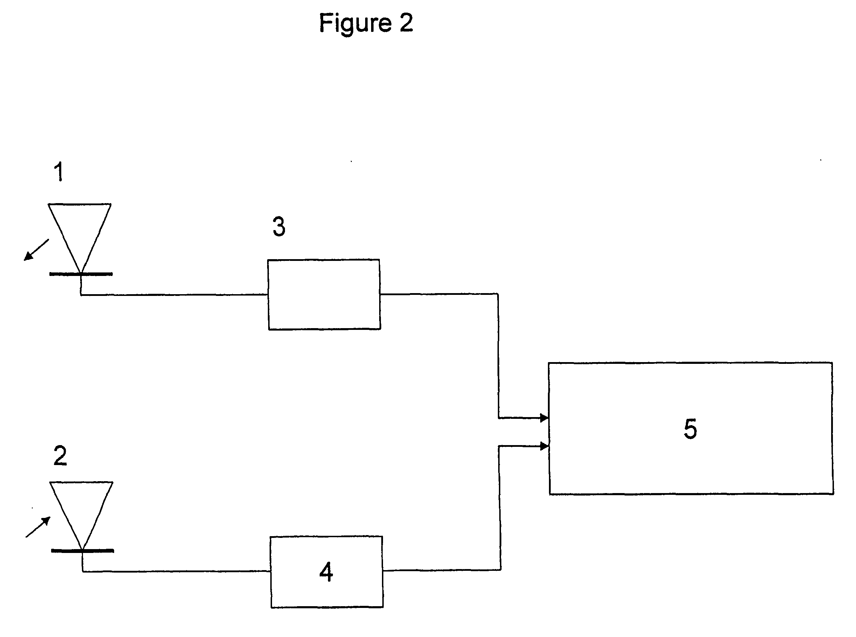

Referring to Figures 1 and 2, the invention comprises a light transmission and detection system including transducers 1, 2 comprising an LED and photo-detector with suitable amplifiers 3, 4 as shown in Figure 2. Once the transducers 1, 2 are attached to the skin the central control unit 5 calibrates them by driving the LED 1 with a voltage appropriate to detect a mid-scale voltage from the photo-detector 2. The photo-detector 2 signals are digitised by A/D1 and A/D2. The drive voltages for the LEDS are produced from the output of D/A1 and D/A2. Once the calibration process is complete the central control unit 5 collects data from the photo-detector 4 (Figure 2) at a sampling rate appropriate for the application. For DVT detection a sample rate of 6 Hz is used. A user input device 6 such as a keypad and a display for output, for example an LCD screen or LED indicators or similar is used. There is also provided an input/output port for PC connection, printer or other form of data logging device.

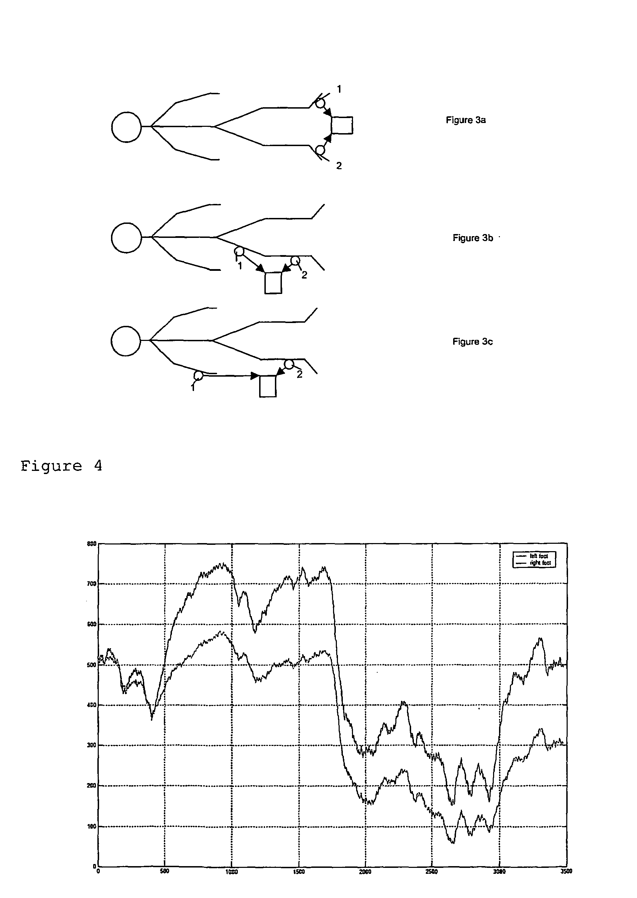

Figures 3a to c show a preferred embodiment of the invention using a two channel system using two transducers 1, 2 for differential signal analysis. For the purpose of DVT detection, the transducers 1, 2 are positioned on the soles of the feet of a patient as shown in Figure 3a. The configuration of 3b can give an indication of the approximate location of DVT. If the vasomotor signals are similar the DVT will be located in the thigh whereas if the vasomotor signals are dissimilar the DVT will be located in the calf. The arrangement in Figure 3c indicates the pulse transit time between the upper and lower extremities and thus an indication of arterial stiffness. Figure 4 shows the signal derived from the soles of the feet of a healthy subject using a two channel system. The signal from each transducer is similar if not identical. The presence of a unilateral DVT is detected by measuring any dissimilarity between the two signals.

The output presented to the user can take the form of a detailed display of vasomotor signals collected from the transducers 1, 2 as shown in Figure 4 to a simple indication of a condition being present or absent. The display can be configured to the application.

The sampling rate of the transducer 1, 2 signals is such that the heart rate component can be resolved to within +/- 1 ms or better if the heart rate is of interest in the assessment being performed, for example in autonomic function testing. Otherwise sampling frequencies that meet the Nyquist requirements are adequate.

The signals acquired from each transducer 1, 2 are subject to appropriate analytical algorithms. The signals are subject to amongst others complex demodulation a mathematical technique used for investigating the vasomotor activity centred at specific frequencies with a bandwidth chosen in accordance with the application, for example DVT detection. The output of the complex demodulation algorithm consists of an amplitude signal and a phase signal which when combined, produce a time varying signal modulated by both amplitude and phase with limited bandwidth, all centred on the demodulating frequency.

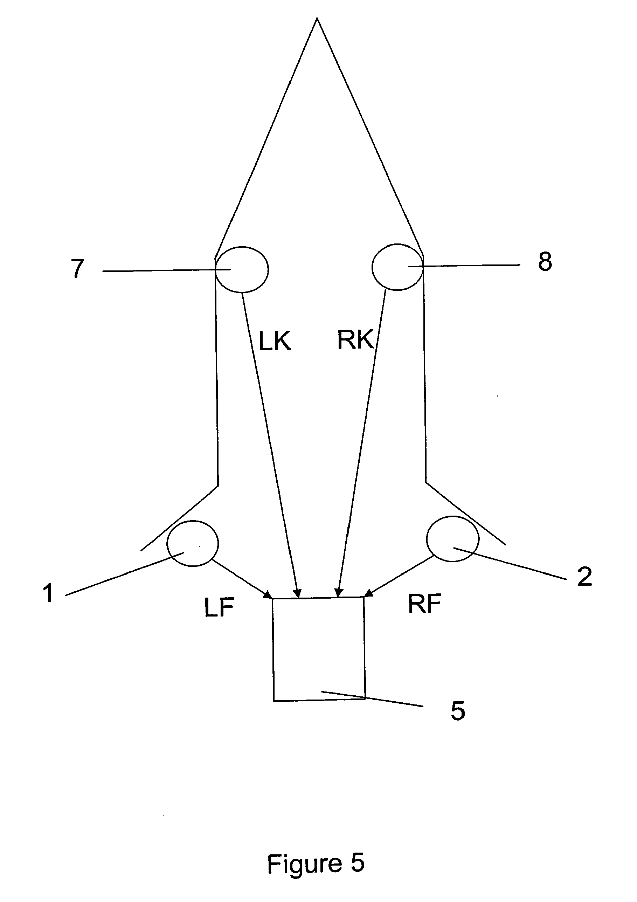

As well as the arrangements shown in Figures 3a to c, another preferred embodiment has two further transducers 7, 8 applied behind the knees for a four channel system as shown in Figure 5. The signals are passed through the stages of signal pre-processing including filtering and DC removal followed by complex demodulation at a set of chosen frequencies, for example 8 to 30 cycles per minute. The mean absolute phase differences (MAPD) from the right foot (RF) and the left foot (LF) are calculated for each frequency to produce a spectrum RFLF(MAPD) and the RFLF(MAPD) is then used by a pattern classifier such as a pre-trained artificial neural network to provide an output on a screen that there is either "DVT PRESENT" or "DVT NOT PRESENT".

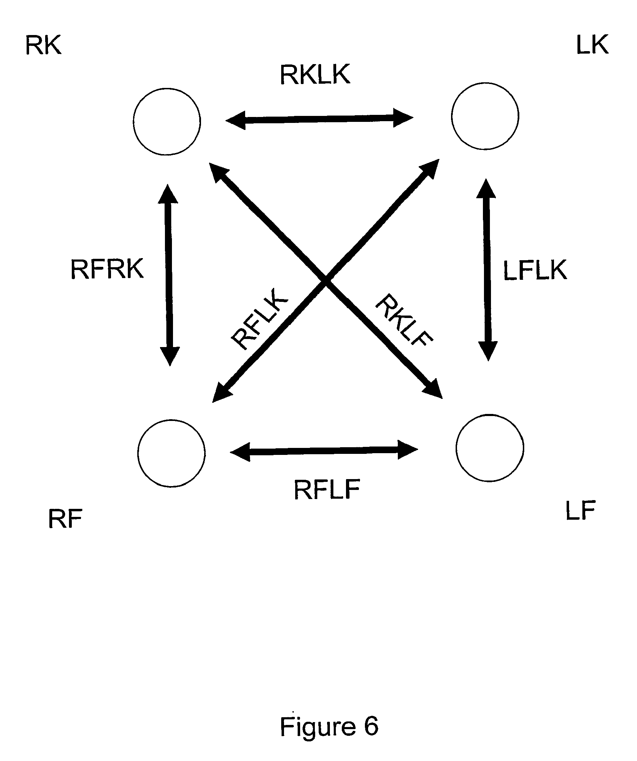

For a four channel system as shown in Figure 5, there will be six MAPDs as shown in Figure 6:-
Right Foot Left Foot : RFLF = mean ( abs ( RF(Φ) LF(Φ)),
Right Knee Left Knee : RKLK = mean ( abs ( RK(Φ) - LK(Φ) ) ),
Right Foot Right Knee : RFRK = mean ( abs ( RF(Φ) - RK(Φ) ) ),
Left Foot Left Knee : LFLK = mean ( abs ( LF(Φ) - LK(Φ) ) ),
Right Foot Left Knee : RFLK = mean ( abs ( RF(Φ) - LK(Φ) ) ),
Right Knee Left Foot : RKLF = mean ( abs ( RK(Φ) - LF(Φ) ) ),
giving six times the diagnostic information of the two channel system, described above.

In addition to detecting DVT, the present invention can monitor and assess a range of clinical conditions including diabetic peripheral neuropathy, critical limb ischaemia, autonomic neural function and arterial and venous disease.

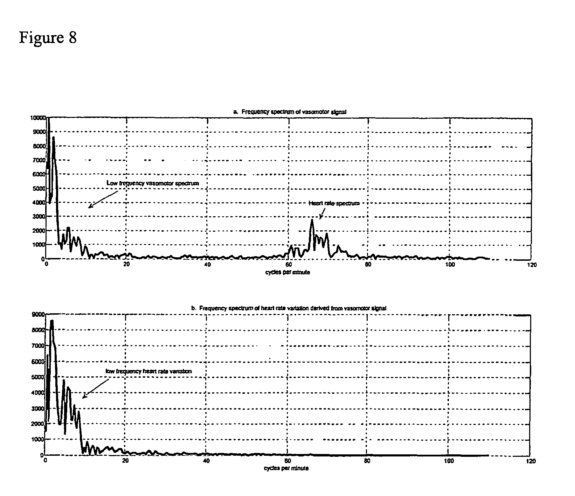

In each of these conditions the vasomotor activity of the micro circulation possesses a unique signature which is extracted and assessed using the appropriate signal processing algorithms. These algorithms are tuned to the appropriate frequency bands determined by the clinical condition of interest. The algorithms exploit the property of vasomotor symmetry between the left and right feet and also use the similarity between the low frequency components of the vasomotor activity and the low frequency components of heart rate variation. As shown in Figure 8, the device according to the invention, extracts from the vasomotor signal the heart rate variation and direct comparison of the simultaneous low frequency heart rate variation and the low frequency vasomotor variation provides information relating to diabetic sympathetic neuropathy, any dissimilarity between the two components indicating diabetic sympathetic neuropathy.

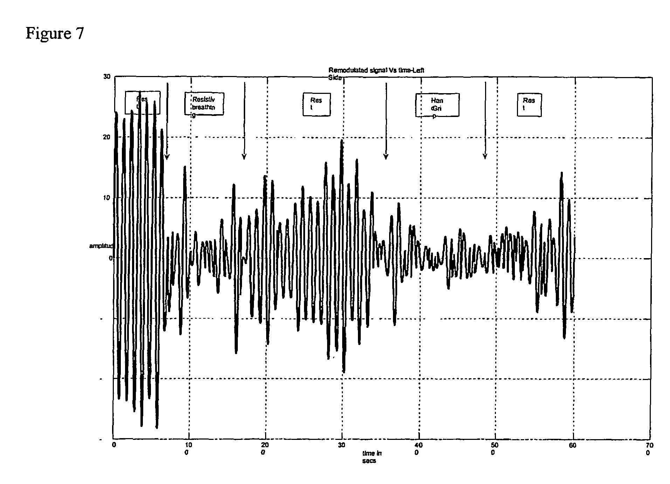

Figure 7 shows the changes in vasomotor activity related to increased breathing resistance and the hand grip test of a healthy person. These tests affect systemic blood pressure and cardiac output which in turn cause neurologically mediated responses in heart rate and peripheral vasomotor activity as observed with the transducers on the soles of the feet. Any changes from the signals in Figure 7 between the resting phase and the increased breathing resistance and the hand grip test will indicate diabetic sympathetic neuropathy since the pathology of the sympathetic nerve fibres which innovate the micro-blood vessels within the feet will cause significant change in vasomotor behaviour.

## Claims

1. A device comprising a light transmission and detection system to assess vasomotor activity at individual sites on a body for the monitoring and assessment of a range of clinical conditions including suspected DVT and diabetic peripheral neuropathy **characterised in that** the device includes one or more wave transducers (1,2,7,8), the wave transducers (1,2,7,8) placed at several sites on a body, control means (4,5) to continuously measure the light absorbed at the sites and provide signals relating to the differential value between the sites.

2. A device as claimed in claim 1, **characterised in that** the control means (4,5) measure the light reflected at the sites and provide signals relating to the differential value between the sites.

3. A device as claimed in claims 1, or 2 **characterised in that** the signals relating to the simultaneous low frequency heart rate variation and the low frequency vasomotor variation are extracted and compared.

4. A method for indicating the presence or absence of DVT, by means of a device as claimed in claims 1 or 2, **characterised in that** a wave transducer (1,2) is placed on each sole of the feet on a body, the control means (4,5) providing signals relating to the differential value between the soles.

5. A method for indicating the location on the leg, of any DVT, by means of a device as claimed in claims 1, or 2, **characterised in that** a wave transducer (1,2) is placed one on the thigh and one on the foot of a leg on a body, the control means (4,5) providing signals relating to the differential value between the sites.

6. A method for indicating the presence or absence of arterial stiffness by means of a device as claimed in claims 1 or 2, **characterised in that** a wave transducer (1,2) is placed one on an upper extremity and one on a lower extremity on a body, the control means (4,5) providing signals relating to the differential value between the upper and lower extremities.

7. A method for detecting vasomotor activity at the sites by means of a device as claimed in claims 1 or 2, **characterised in that** wave transducers (1,2,7,8) are placed one behind each knee and one on each foot on a body, the control means providing signals relating to the vasomotor activity at the sites.

8. A method as claimed in claim 7 **characterised in that**, the control means (4,5) provides signals relating to the vasomotor activity variation between the sites.

9. A method as claimed in claim 7 or 8 **characterised in that**, the control means (4,5) provides signals relating to the heart rate variation at the sites.

10. A method as claimed in claim 9 **characterised in that**, the control means (4,5) provides signals relating to the differences in the heart rate variation between the sites.

## Patentansprüche

1. Vorrichtung, die ein Lichttransmissions- und Erfassungssystem umfasst zum Beurteilen vasomotorischer Aktivität an individuellen Stellen an einem Körper für die Überwachung und Beurteilung eines Bereiches klinischer Zustände einschließlich vermuteter DVT und diabetischer peripherer Neuropathie, **dadurch gekennzeichnet, dass** die Vorrichtung einen oder mehrere Wellenwandler (1, 2, 7, 8), wobei die Wellenwandler (1, 2, 7, 8) an mehreren Stellen an einem Körper angeordnet werden, und Steuerungsmittel (4, 5) zum kontinuierlichen Messen des Lichts, das an den Stellen adsorbiert wird, und zum Bereitstellen von Signalen, die den Differenzwert zwischen den Stellen betreffen, einschließt.

2. Vorrichtung gemäß Anspruch 1, **dadurch gekennzeichnet, dass** die Steuerungsmittel (4, 5) das Licht messen, das an den Stellen reflektiert wird, und Signale bereitstellen, die den Differenzwert zwischen den Stellen betreffen.

3. Vorrichtung gemäß Anspruch 1 oder 2, **dadurch gekennzeichnet, dass** die Signale, welche die gleichzeitige niederfrequente Herzfrequenzvariation und die niederfrequente vasomotorische Variation betreffen, extrahiert und verglichen werden.

4. Verfahren zum Angeben des Vorhandenseins oder der Abwesenheit von DVT mittels einer Vorrichtung gemäß Anspruch 1 oder 2, **dadurch gekennzeichnet, dass** ein Wellenwandler (1, 2) auf jeder Sohle des Fußes an einem Körper angeordnet wird, wobei die Steuerungsmittel (4, 5) Signale bereitstellen, welche den Differenzwert zwischen den Sohlen betreffen.

5. Verfahren zum Angeben des Orts an dem Bein von jeglicher DVT mittels einer Vorrichtung gemäß Anspruch 1 oder 2, **dadurch gekennzeichnet, dass** ein Wellenwandler (1, 2), einer an dem Schenkel und einer an dem Fuß eines Beins, an einem Körper angeordnet wird, wobei die Steuerungsmittel (4, 5) Signale bereitstellen, welche den Differenzwert zwischen den Stellen betreffen.

6. Verfahren zum Angeben des Vorhandenseins oder der Abwesenheit von arterieller Verhärtung mittels einer Vorrichtung gemäß Anspruch 1 oder 2, **dadurch gekennzeichnet, dass** ein Wellenwandler (1, 2), einer an einer oberen Extremität und einer an einer unteren Extremität, an einem Körper angeordnet wird, wobei die Steuerungsmittel (4, 5) Signale bereitstellen, welche den Differenzwert zwischen den oberen und unteren Extremitäten betreffen.

7. Verfahren zur Erfassung vasomotorischer Aktivität an den Stellen mittels einer Vorrichtung gemäß Anspruch 1 oder 2, **dadurch gekennzeichnet, dass** Wellenwandler (1, 2, 7, 8) einer hinter jedem Knie und einer an jedem Fuß an einem Körper angeordnet werden, wobei die Steuerungsmittel Signale bereitstellen, welche die vasomotorische Aktivität an den Stellen betreffen.

8. Verfahren gemäß Anspruch 7, **dadurch gekennzeichnet, dass** die Steuerungsmittel (4, 5) Signale bereitstellen, welche die Variation der vasomotorischen Aktivität zwischen den Stellen betreffen.

9. Verfahren gemäß Anspruch 7 oder 8, **dadurch gekennzeichnet, dass** die Steuerungsmittel (4, 5) Signale bereitstellen, welche die Herzfrequenzvariation an den Stellen betreffen.

10. Verfahren gemäß Anspruch 9, **dadurch gekennzeichnet, dass** die Steuerungsmittel (4, 5) Signale bereitstellen, welche die Differenzen der Herzfrequenzvariation zwischen den Stellen betreffen.

## Revendications

1. Dispositif comprenant un système de transmission et de détection de lumière destiné à évaluer une activité vasomotrice au niveau de sites individuels sur un corps pour le suivi et l'évaluation d'une plage de conditions cliniques comportant une DVT et une neuropathie périphérique diabétique soupçonnées, **caractérisé en ce que** le dispositif comporte un ou plusieurs transducteurs d'ondes (1, 2, 7, 8), les transducteurs d'ondes (1, 2, 7, 8) étant placés au niveau de plusieurs sites sur un corps, un moyen de commande (4, 5) pour mesurer continuellement la lumière absorbée au niveau des sites et fournir des signaux relatifs à la valeur différentielle entre les sites.

2. Dispositif tel que revendiqué dans la revendication 1, **caractérisé en ce que** le moyen de commande (4, 5) mesure la lumière réfléchie au niveau des sites et fournit des signaux relatifs à la valeur différentielle entre les sites.

3. Dispositif tel que revendiqué dans les revendication 1 ou 2, **caractérisé en ce que** les signaux relatifs à la variation du rythme cardiaque à basse fréquence et à la variation vasomotrice à basse fréquence, qui se produisent de manière simultanée, sont extraits et comparés.

4. Procédé permettant d'indiquer la présence ou l'absence d'une DVT, au moyen d'un dispositif tel que revendiqué dans les revendications 1 ou 2, **caractérisé en ce qu'**un transducteur d'ondes (1, 2) est placé sur chaque plante des pieds d'un corps, le moyen de commande (4, 5) fournissant des signaux relatifs à la valeur différentielle entre les plantes des pieds.

5. Procédé permettant d'indiquer l'emplacement sur la jambe, d'une DVT, au moyen d'un dispositif tel que revendiqué dans les revendications 1 ou 2, **caractérisé en ce que** l'un des transducteurs d'ondes (1, 2) est placé sur la cuisse et l'autre sur le pied d'une jambe sur un corps, le moyen de commande (4, 5) fournissant des signaux relatifs à la valeur différentielle entre les sites.

6. Procédé permettant d'indiquer la présence ou l'absence d'une rigidité artérielle au moyen d'un dispositif tel que revendiqué dans la revendication 1 ou 2, **caractérisé en ce que** l'un des transducteurs d'ondes (1, 2) est placé sur une extrémité supérieure et l'autre sur une extrémité inférieure d'un corps, le moyen de commande (4, 5) fournissant des signaux relatifs à la valeur différentielle entre les extrémités supérieure et inférieure.

7. Procédé permettant de détecter une activité vasomotrice au niveau des sites au moyen d'un dispositif tel que revendiqué dans la revendication 1 ou 2, **caractérisé en ce que** les transducteurs d'ondes (1, 2, 7, 8) sont placés l'un derrière chaque genou et l'autre sur chaque pied d'un corps, le moyen de commande fournissant des signaux relatifs à l'activité vasomotrice au niveau des sites.

8. Procédé tel que revendiqué dans la revendication 7, **caractérisé en ce que** le moyen de commande (4, 5) fournit des signaux relatifs à la variation de l'activité vasomotrice entre les sites.

9. Procédé tel que revendiqué dans la revendication 7 ou 8, **caractérisé en ce que** le moyen de commande (4, 5) fournit des signaux relatifs à la variation du rythme cardiaque au niveau des sites.

10. Procédé tel que revendiqué dans la revendication 9, **caractérisé en ce que** le moyen de commande (4, 5) fournit des signaux relatifs aux différences de la variation du rythme cardiaque entre les sites.
